(19)

Europäisches
Patentamt
European
Patent Office
Office européen
des brevets

(11) **EP 4 246 523 A1**

(12) **EUROPEAN PATENT APPLICATION**

(43) Date of publication:
**20.09.2023 Bulletin 2023/38**

(21) Application number: **22162590.8**

(22) Date of filing: **16.03.2022**

(51) International Patent Classification (IPC):
***G16H 10/20*** (2018.01)　　***G16H 50/20*** (2018.01)

(52) Cooperative Patent Classification (CPC):
**G16H 50/20; G16H 10/20**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **National University of Ireland Galway
Galway (IE)**

(72) Inventors:
• **STAUNTON, Brendan
  Dublin 18, D18T2W2 (IE)**
• **CUNDIFF, Geoffrey
  North Vancouver, BC V7N1E6 (CA)**

(74) Representative: **Barker Brettell LLP
100 Hagley Road
Edgbaston
Birmingham B16 8QQ (GB)**

(54) **SYSTEM AND METHOD FOR PELVIC FLOOR DYSFUNCTION DETERMINATION**

(57)　　An apparatus (100) for determining a pelvic floor dysfunction condition of a patient is provided. The apparatus (100) comprises a processor (105). The processor (105) is configured to receive patient data. The patient data comprises at least one of questionnaire data and behavioural data of the patient. The processor (105) is also configured to provide, using a machine learning algorithm (115), a determination of one or more pelvic floor dysfunction conditions of the patient based on received patient data.

200

202 ┌─────────────────────────────┐
     │     Obtain patient data     │
     └─────────────────────────────┘
                    │
                    ▼
204 ┌─────────────────────────────┐
     │  Determine one or more pelvic floor  │
     │   dysfunction conditions of patient  │
     │  using machine learning algorithm    │
     └─────────────────────────────┘

FIGURE 2

EP 4 246 523 A1

**Description**

TECHNICAL FIELD

The present invention relates to an apparatus for and method of determining pelvic floor dysfunction of a patient.

BACKGROUND

Diagnosis of pelvic floor and bladder dysfunction is a complex process. Different pelvic floor and bladder conditions have a variety of overlapping symptoms and a variety of overlapping causes. For example, two patients can present with the same symptoms (e.g., episodes of incontinence) but the underlying causes of those symptoms can be different (e.g., one patient may have Overactive Bladder, OAB, whilst the other patient may have Stress Urinary Incontinence, SUI).

A correct diagnosis is imperative, as treatment for OAB would not be effective for SUI-related incontinence, and similarly treatment for SUI would not be effective for OAB-related incontinence.

Current methods for diagnosing patients with pelvic floor and bladder dysfunction are lengthy and complicated, with a series of trial-and-error treatment approaches until a suitable outcome is achieved. That results in longer treatment periods, increased cost of treatment, reduced treatment safety and efficacy, and patient frustration.

The time between a patient first presenting with symptoms and being correctly diagnosed is typically several months and in some cases years. Early diagnosis can lead to better outcomes as many pelvic floor and bladder conditions have an early intervention treatment window which can help to halt progression of the condition. Correct diagnosis is therefore imperative for effective and timely treatment.

However, the identification and diagnosis of pelvic floor and bladder dysfunction presents a daunting problem in terms of accuracy, consistency and efficiency.

The present invention has been devised with the foregoing in mind.

SUMMARY

According to a first aspect, there is provided an apparatus for determining a pelvic floor dysfunction condition of a patient. The apparatus may comprise a processor. The processor may be configured to receive patient data. The patient data may be or comprise at least one of questionnaire data and behavioural data of the patient. The processor may also be configured to provide, using a machine learning algorithm, a determination of one or more pelvic floor dysfunction conditions of the patient based on received patient data.

Known approaches for identifying and determining such conditions are time-consuming and resource intensive, requiring patients to answer a large number of questions, undergo extensive data recording exercises or undergo invasive diagnostic procedures. In addition, known approaches for identifying and determining such conditions have low accuracy and consistency, as patients often present a vast range of variation that can be difficult to capture and classify.

The apparatus may enable provision of efficient, accurate and reliable determination of one or more pelvic floor dysfunction conditions or bladder conditions of a patient, compared to the slow, inefficient and often inaccurate conventional approach of manually assessing questionnaire data alone. The apparatus may also provide inherent validation of questionnaire data (which can be subjective) and/or behavioural data of the patient using population-based data, by virtue of the machine learning algorithm being trained using data and corresponding diagnoses of previous patients. That may further increase the accuracy and reliability of the determination. The population-based data may enable the machine learning algorithm to be sensitive to the variation patients may present with.

The efficient determination may also enable the apparatus to provide repeat determinations more easily and quickly at different points in time based on new, different or changing patient data. That may enable a physician to more quickly determine whether an existing condition determination is correct and/or whether a current treatment plan is improving the patient's symptoms. That may reduce a time between a patient first presenting with symptoms and being correctly diagnosed, which may help to halt progression of the pelvic floor dysfunction condition or bladder condition and improve patient health.

The machine learning algorithm may be or comprise a neural network. The neural network may comprise an input layer comprising a plurality of neurons. Each neuron may correspond to a different possible question answer or metric of behavioural data.

That may enable the apparatus to provide a determination of one or more conditions based on incomplete data or different subsets of data. For example, the apparatus may be able to provide a determination based on questionnaire data, behavioural data, or both, using the same underlying approach. That may greatly increase the flexibility and applicability of the apparatus in comparison to conventional diagnostic approaches for such conditions. That may also enable potentially subjective questionnaire data of the patient to be validated using behavioural data of the patient, further increasing accuracy and efficiency of the determination.

The processor may be configured to provide an initial determination of one or more conditions based on questionnaire data received at a first time. The processor may also be configured to provide an updated or validating determination of one or more conditions based on at least behavioural data received at a second time later than the first time. The updated or validating determination may be based on both behavioural data and questionnaire data received at the second time.

The flexibility of the apparatus to efficiently provide an accurate determination based on questionnaire data and/or behavioural data of the patient may enable the apparatus to provide repeat determinations more easily and quickly at different points in time based on new, different or changing patient data. That may enable a physician to quickly determine whether an existing condition determination is correct and/or whether a current treatment plan is improving the patient's symptoms.

The processor may be further configured to train or update the machine learning algorithm based on the received patient data.

Training or updating the algorithm based on received patient data may enable increased reliability and accuracy of the determination, by increasing the size of the training dataset for the machine learning algorithm over time. In that way, the machine learning algorithm may be substantially continually updated to take into account population outcomes, rather than relying on a fixed approach as with conventional diagnostic approaches.

The apparatus may further comprise a memory configured to store a plurality of questions. The plurality of questions may relate to a plurality of different conditions. The processor may be further configured to select questions relating to one or more conditions for presentation to a user. The processor may be configured to select a next question based on patient data received in response to one or more preceding questions.

Known approaches for identifying and determining such pelvic floor dysfunction conditions or bladder conditions include lengthy, condition-specific questionnaires. However, patients may present with one or more of several possible conditions, and so reliance on one subset of questions may result in reduced sensitivity and specificity in determining the patient's condition.

By selecting a next question, from a plurality of questions relating to a plurality of different conditions, based on patient data received in response to one or more preceding questions, the apparatus may extract the most clinically relevant data more efficiently from the questionnaire data. The clinically relevant data may include questionnaire data relating to a plurality of conditions, whilst minimizing a number of questions presented to the patient. Irrelevant questionnaire data can also be reduced or eliminated. That may reduce a burden on both the patient and physician. In addition, only relevant questionnaire data may be provided to the machine learning algorithm, further improving efficiency and accuracy of the determination of one or more conditions.

The memory may be configured to store a ranking for the plurality of different conditions in order of condition prevalence. The processor may be configured to select a first question relating to a most prevalent condition.

The processor may be configured to determine if patient data received in response to one or more preceding questions is indicative of a condition associated with the one or more preceding questions. If the patient data is not indicative of a condition associated with the one or more preceding questions, the processor may be configured to select a next question relating to a next most prevalent or likely condition.

That may result in only the most clinically relevant data being extracted from the questions and provided to the machine learning algorithm, thereby further improving efficiency and accuracy of the determination of one or more conditions.

The processor may be configured to use branching logic to select a next question. The branching logic may be pre-determined or fixed.

The behavioural data may comprise one or more of a volume of fluid drunk, a volume of fluid urinated, an amount of caffeine consumed, an amount of alcohol consumed, a number of incontinence episodes, a number of nocturia episodes, a number of bladder voids and a number of incontinence episodes associated with urgency.

The behavioural data may be provided in respect of at least one time period. Each time period may be 24 hours. The behavioural data may be or comprise an average of behavioural data provided for a plurality of time periods.

The processor may be configured to generate a digital user interface for a user electronic device. The digital user interface may enable input of patient data to the apparatus. That may reduce or minimize anxiety for patients who are embarrassed about their condition. That may also increase patient engagement thereby improving patient health.

The processor may be configured to implement the determination of one or more conditions on a web application on a user electronic device, through a server back-end or in a native application on a user electronic device.

The processor may be configured to determine a targeted treatment program based on a determination of the one or more conditions by the processor. That may enable treatment to be substantially personalised to the patient. The processor may further be configured to determine an updated targeted treatment program based on an updated determination of the one or more conditions by the processor. That may enable a treatment approach to be quickly and easily updated if a previous treatment program has not had the desired effect on improving a patient's condition(s).

According to a second aspect, there is provided a method of determining a pelvic floor dysfunction condition of a patient. The method may comprise obtaining or receiving patient data. The patient data may be or comprise at least one of questionnaire data and behavioural data of the patient. The method may also comprise providing, using a machine learning algorithm, a determination of one or more pelvic floor dysfunction conditions of the patient based on received patient data.

The method of the second aspect may be performed on or using the apparatus of the first aspect. The method of the second aspect may comprise one or more optional features corresponding to optional features of the apparatus of the first aspect.

According to a third aspect, there is provided a non-transitory computer program comprising instructions which, when the program is executed by a processor, cause the processor carry out the method of second aspect.

According to a fourth aspect, there is provided a computer-readable medium having the computer program of the third aspect stored thereon.

Features which are described in the context of separate aspects and embodiments of the present invention may be used together and/or be interchangeable wherever possible. Similarly, where features are described in the context of a single embodiment for brevity, those features may also be provided separately or in any sub-combination. Features described in connection with the apparatus of the first aspect may have corresponding features definable with respect to the method of the second aspect, computer program of the third aspect or computer-readable medium of the third aspect, and vice versa, and those embodiments are specifically envisaged.

BRIEF DESCRIPTION OF THE DRAWINGS

The present invention will now be described, by way of example only, with reference to the accompanying drawings in which:

FIG. 1 shows an embodiment of an apparatus for determining one or more pelvic floor dysfunction conditions or bladder conditions of a patient in accordance with the invention;

FIG. 2 shows an embodiment of a method for determining one or more pelvic floor dysfunction conditions or bladder conditions of a patient in accordance with the invention;

FIG. 3 shows another embodiment of an apparatus for determining one or more pelvic floor dysfunction conditions or bladder conditions of a patient, comprising a memory configured to store a plurality of questions in accordance with the invention;

FIGs. 4A to 4F show a flowchart illustrating how the processor of the apparatus shown in FIG. 3 is configured to select questions from the plurality of questions in the memory;

FIG. 5 shows a neural network used by the processor of the apparatus shown in FIG. 3 to provide a determination of one or more pelvic floor dysfunction conditions or bladder conditions;

FIG. 6 shows another embodiment of a method for determining one or more pelvic floor dysfunction conditions or bladder conditions of a patient using the apparatus shown in FIG. 3; and

FIGs. 7A and 7B respectively show a conventional diagnostic and treatment pathway, and a determination and treatment pathway in accordance with the invention.

Like reference numbers and designations in the various drawings indicate like elements.

DETAILED DESCRIPTION

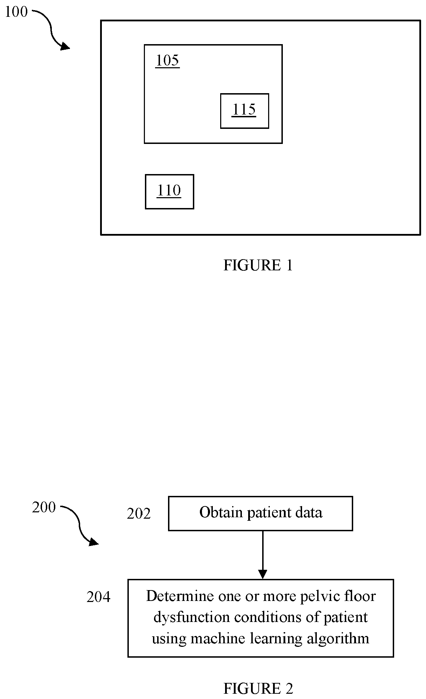

Figure 1 shows an apparatus 100 for determining a pelvic floor dysfunction condition of a patient, in accordance with an embodiment of the present invention. The apparatus 100 comprises a processor 105. The processor 105 is configured to receive patient data, in particular questionnaire data relating to one or more potential pelvic floor dysfunction conditions of the patient, and behavioural data of the patient. In the embodiment shown, the apparatus 100 comprises a data reception module 110 from which the patient data is received by the processor 105, although that is not essential. The processor 105 is further configured to determine one or more pelvic floor dysfunction conditions of the patient based on received patient data using a machine learning (ML) algorithm 115. In the embodiment shown, the ML algorithm 115 is or comprises a neural network, although that is not essential.

The processor 105 may be configured to implement the determination of the one or more pelvic floor dysfunction conditions on a web application on a user electronic device (for example, a personal computer or smartphone), through a server back-end or in a native application on a user electronic device. The apparatus 100 may also be configured to generate a digital user interface for a user electronic device. The digital user interface may enable user input of patient data to the apparatus 100 (for example, via a keyboard or a touch screen) and/or may display an output of a determination of one or more pelvic floor dysfunction conditions by the processor 105.

Figure 2 shows a method 200 of determining a pelvic floor dysfunction condition of a patient, in accordance with an embodiment of the present invention. The method 200 may be carried out using the apparatus 100 described above.

Step 202 of the method 200 comprises obtaining patient data, in particularly questionnaire data relating to one or more potential pelvic floor dysfunction conditions of the patient and behavioural data of the patient. Step 204 of the method 200 comprises providing, using a machine learning algorithm, a determination of one or more pelvic floor dysfunction conditions of the patient.

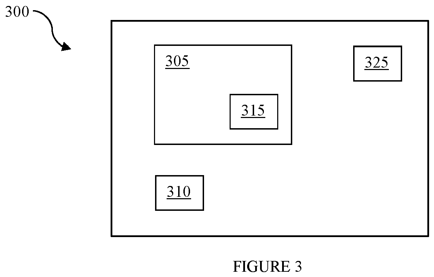

Figure 3 shows apparatus 300 for determining a pelvic floor dysfunction condition of a patient, in accordance with another embodiment of the present invention. The apparatus 300 comprises a processor 305 and a machine learning algorithm 315 as described above in respect of the apparatus 100 shown in Figure 1, with like reference numerals indicating like elements.

In the embodiment shown, the apparatus 300 further comprises a memory 325. The memory 325 is configured to store a plurality of questions relating to a plurality of different pelvic floor dysfunction conditions. The processor 305 is configured to select questions from the memory 325 relating to one or more conditions for presentation to a user (for example, via a digital user interface generated by the apparatus 300, as described above), in order to obtain questionnaire data relating to one or more pelvic floor dysfunction conditions of the patient.

Current diagnostic methods for pelvic floor dysfunction conditions utilise condition-specific questionnaires. The clinical results of such condition-specific questionnaires may be less than ideal as patients may have one or more of several possible conditions. Reliance on one subset of questions from a questionnaire may result in sub-optimal sensitivity and specificity of the tested pelvic floor/bladder dysfunction. Furthermore, many patients have two or more related conditions. With each questionnaire being condition-specific, a patient presenting with multiple conditions may be required to take multiple questionnaires. The evaluation of a patient using multiple condition-specific questionnaires may therefore be lengthy, inconvenient and logistically challenging.

The memory 325 is configured to store a ranking for the plurality of conditions in order of condition prevalence. The processor 305 is configured to select a first question relating to a most prevalent condition (for example, stress urinary incontinence, SUI).

The plurality of questions stored in the memory 325 relates to a plurality of different conditions. The questions comprise clinically validated condition-specific questions relating to inter alia quality of life, symptoms, bother index and impact on quality of life. The questions may be selected from one or more conventional clinically validated condition-specific questionnaires, for example MESA (Medical, Epidemiologic and Social aspects of Aging urinary incontinence questionnaire), POPDI (Pelvic Organ Prolapse Distress Inventory), PFDI (Pelvic Floor Distress Inventory), PGI-I (Patient Global Impression of Improvement), ICIQ-OAB (International Consultation on Incontinence Questionnaire Overactive Bladder), ICIQ-OAB-QOL (International Consultation on Incontinence Questionnaire Overactive Bladder Quality of Life), QUID (Questionnaire for Urinary Incontinence Diagnosis), SARC-F (Strength, Ambulation, Rising from a chair, stair Climbing and history of Falling) etc.

However, rather than present every question relating to each of the plurality of conditions to a user to obtain patient data, in the embodiment shown the processor 305 is configured to select a next question based on patient data received in response to one or more preceding questions. The processor 305 is configured to determine if patient data received in response to one or more preceding questions is indicative of a condition associated with the one or more preceding questions. If the patient data is not indicative of a condition associated with the one or more preceding questions, the processor 305 is configured to select a next question relating to one or more next most likely conditions. The next most likely condition(s) may be the next most prevalent condition(s), which may allow the most likely condition(s) to be assessed first in order to avoid obtaining potentially irrelevant data, although that is not essential.

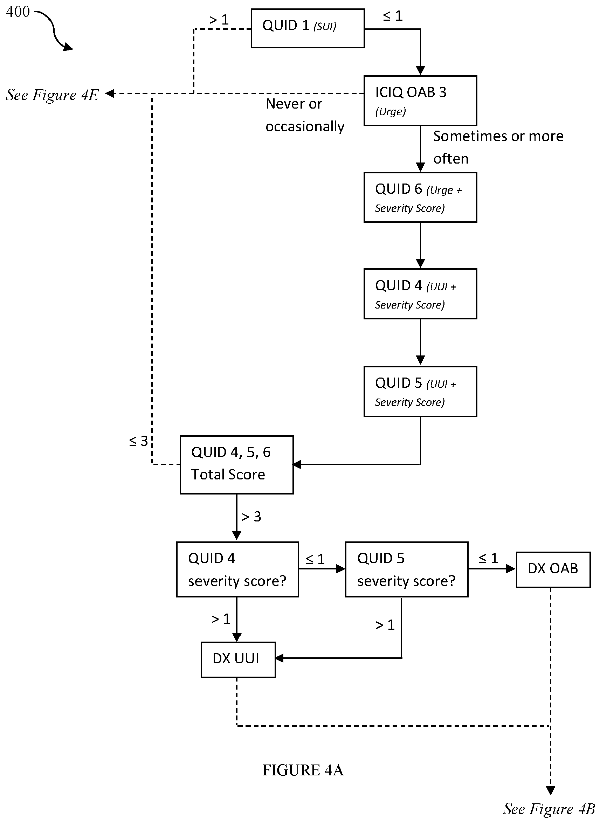

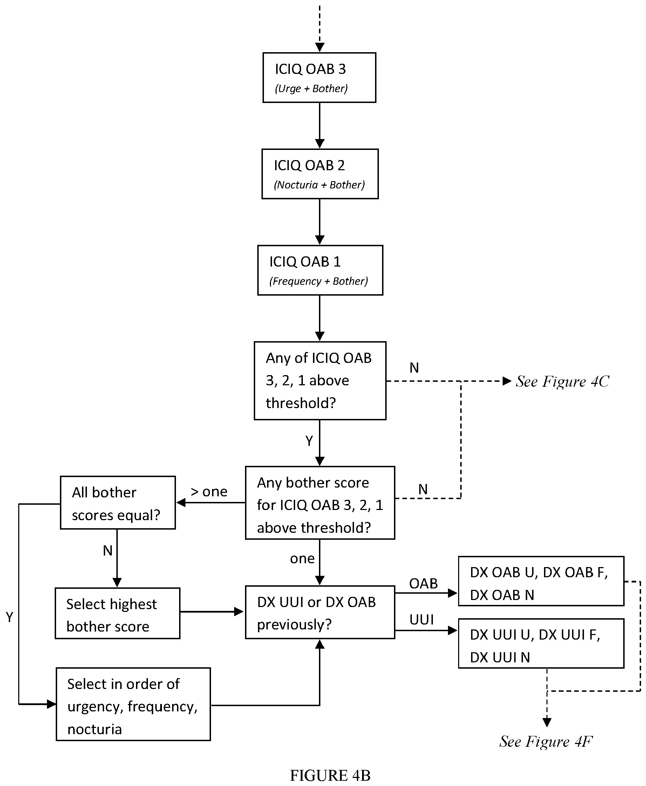

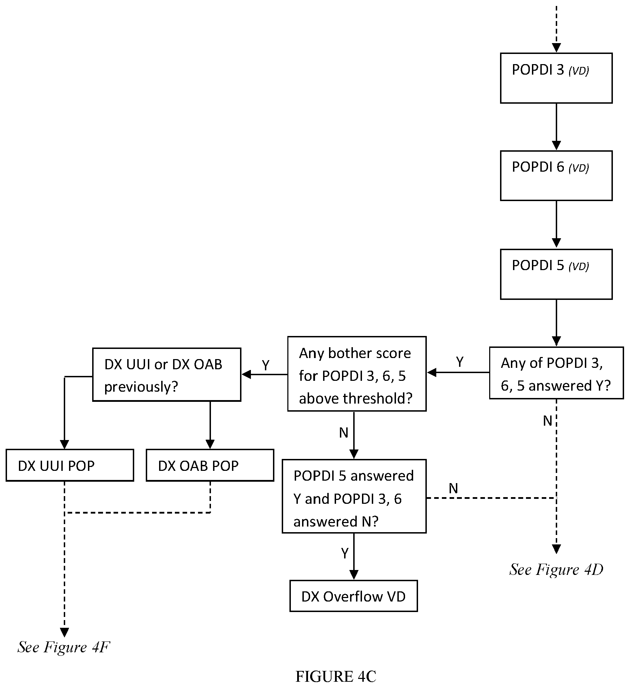

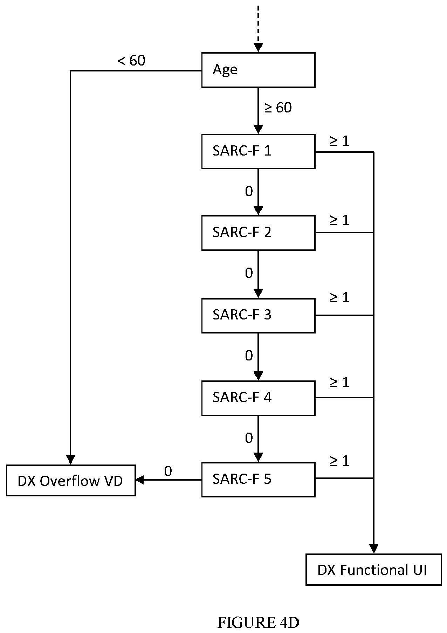

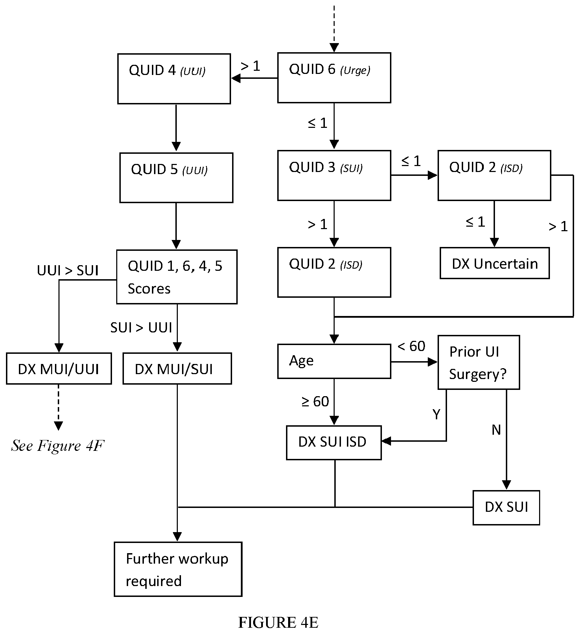

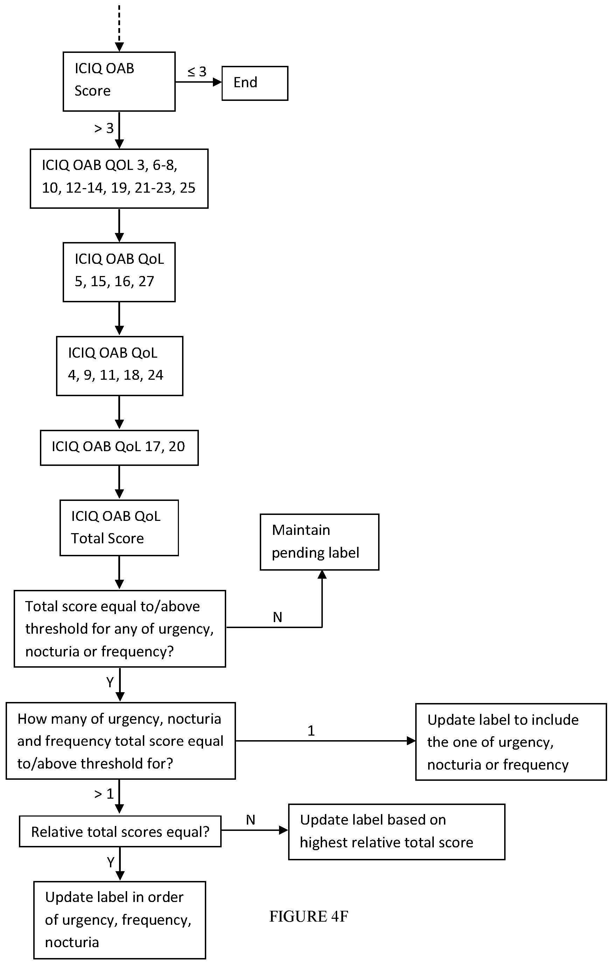

Figure 4 shows a flowchart or algorithm 400 illustrating an example of how the processor 305 may be configured to select a next question based on patient data received in response to one or more preceding questions.

The questions which may be selected in the flowchart 400 and the clinically validated questionnaires from which they are taken are shown below. It will be appreciated that other questions from the same or other clinically validated questionnaires relating to pelvic floor dysfunction conditions and/or bladder conditions may be stored in the memory 325, in addition or alternatively to the questions shown in the flowchart 400.

MESA *(Condition Indicated):*

1. Does coughing gently cause you to lose urine? *(SUI)*
2. Does coughing hard cause you to lose urine? *(SUI)*
3. Does sneezing cause you to lose urine? *(SUI)*
4. Does lifting cause you to lose urine? *(SUI)*
5. Does bending cause you to lose urine? *(ISD)*
6. Does laughing cause you to lose urine? *(MUI)*
7. Does walking briskly/jogging cause you to lose urine? *(ISD)*
8. Does straining when constipated cause you to lose urine? *(SUI)*
9. Does getting up from a standing position cause you to lose urine? *(ISD MUI)*
10. Some people receive very little warning and suddenly find that they are losing, or about to lose, urine beyond their control? How often does this happen to you? Would say Never, Rarely, Sometimes, Often? *(UUI)*
11. If you can't find a toilet or find a toilet that is occupied and you have an urge to urinate, how often do you end up losing urine and wetting yourself? Would you say Never, Rarely, Sometimes, Often? *(UUI)*
12. Do you lose urine when you suddenly have the feeling that your bladder is very full? *(UUI)*
13. Does washing your hands cause you to lose urine? *(UUI)*
14. Does cold weather cause you to lose urine? *(UUI)*
15. Does drinking cold beverages cause you to lose urine? *(UUI)*

POPDI:

1. Do you usually experience pressure in the lower abdomen? If yes, how much does this bother you (0-4)?
2. Do you usually experience heaviness or dullness in the pelvic area? If yes, how much does this bother you (0-4)?
3. Do you usually have a bulge or something falling out that you can see or feel in the vaginal area? If yes, how much does this bother you (0-4)?
4. Do you usually have to push on the vagina or around the rectum to have or complete a bowel movement? If yes, how much does this bother you (0-4)?
5. Do you usually experience a feeling of incomplete bladder emptying? If yes, how much does this bother you (0-4)?
6. Do you ever have to push up on a bulge in the vaginal area with your fingers to start or complete urination? If yes, how much does this bother you (0-4)?
19. Do you usually experience difficulty emptying your bladder? If yes, how much does this bother you (0-4)?

PGI-I:

1. Check the number/option that best describes how your post treatment condition is now compared to how it was before treatment (Very much better; Much better; Little better; No change; Little worse; Much worse; Very much worse).

ICIQ-OAB:

1. How often do you pass urine during the day? 1-6, 7-8, 9-10, 11-12, 13 or more? How much does this bother you? Please ring a number between 0 (not at all) and 10 (a great deal).
2. During the night, how many times do you have to get up to urinate, on average? None, 1, 2, 3, 4 or more? How much does this bother you? Please ring a number between 0 (not at all) and 10 (a great deal).
3. Do you have to rush to the toilet to urinate? Never, occasionally, sometimes, most of the time, all of the time? How much does this bother you? Please ring a number between 0 (not at all) and 10 (a great deal).
4. Does urine leak before you can get to the toiler? Never, occasionally, sometimes, most of the time, all of the time? How much does this bother you? Please ring a number between 0 (not at all) and 10 (a great deal).

ICIQ-OAB-QoL *(Condition Indicated)*:

3. Made you carefully plan your journey?
4. Caused you to feel drowsy or sleepy during the day? *(Nocturia)*
5. Caused to plan "escape routes" to toilets in public places? *(Urgency)*
6. Caused you distress?
7. Frustrated you?
8. Made you feel like there is something wrong with you?
9. Interfered with your ability to get a good night's rest? *(Nocturia)*
10. Caused you to decrease your physical activities?
11. Prevented you from feeling rested upon waking in the morning? *(Nocturia)*
12. Frustrated your family and friends?
13. Caused you anxiety or worry?
14. Caused you to stay home more often than you would prefer?
15. Caused you to adjust your travel plans so that you are always near a toilet? *(Urgency)*
16. Made you avoid activities away from toilets (i.e., walks, running, hiking)? *(Urgency)*
17. Made you frustrated or annoyed about the amount of time you spend in the toilet? *(Frequency)*
18. Awakened you during sleeping? *(Nocturia)*
19. Made you worry about odour or hygiene?
20. Made you uncomfortable while travelling with others because of needing to stop for a toilet? *(Frequency)*
21. Affected your relationships with family and friends?
22. Caused you to decrease participating in social gatherings, such as parties or visits with family or friends?
23. Caused you embarrassment?
24. Interfered with getting the amount of sleep you needed? *(Nocturia)*
25. Caused you to have problems with your partner or spouse?
26. Caused you to plan activities more carefully?
27. Caused you to locate the closest toilet as soon as you arrive at a place you have never been? *(Urgency)*
28. Overall, how much do your urinary symptoms interfere with your quality of life? Please ring a number between 0 (not at all) and 10 (a great deal).

QUID *(Condition Indicated)*:

Do you leak urine (even small drops), wet yourself, or wet your pads or undergarments ...

1. when you cough or sneeze? *(SUI)*
2. when you bend down or lift something up? *(SUI/ISD)*
3. when you walk quickly, jog or exercise? *(SUI)*
4. while you are undressing in order to use the toilet? *(UUI)*
5. Do you get such a strong an uncomfortable need to urinate that you leak urine (even small drops) or wet yourself before reaching the toilet? *(UUI)*
6. Do you have to rush to the bathroom because you get a sudden, strong need to urinate? *(Urgency)* Score each question from 0 to 5, where 0 = none of the time, 1 = rarely, 2 = once in a while, 3 = often, 4 = most of the time and 5 = all of the time. Scores for questions 1, 2 and 3 are summed for a stress score, and scores for questions 4, 5 and 6 are summed for an urge score.

SARC-F:

1. How much difficulty do you have moving across a room?
2. How much difficulty do you have climbing one flight of stairs (10 stairs)?
3. How many times have you fallen in the last year?
4. How much difficulty do you have lifting or carrying 10 lbs. (4.5 kg)?
5. How much difficulty do you have transferring from a chair to a bed?

Score each question from 0 to 2, where 0 = none, 1 = some, and 2 = a lot/use an aid or unable.

In Figure 4A, QUID 1 is asked as an initial question of the algorithm 400. If the answer to QUID 1 is > 1, the next step of the algorithm 400 is shown in Figure 4E, with a dotted line used to indicate the next step is not shown in Figure 4A. If the answer to QUID 1 is ≤ 1, the next question is ICIQ OAB 3. If the answer to ICIQ OAB 3 is never or occasionally, the next step of the algorithm 400 is shown in Figure 4E. If the answer to ICIQ OAB 3 is sometimes or more often than sometimes (e.g., most of the time, all of the time), QUID 6, 4 and 5 are asked, with all three to be answered. A total score for QUID 6, 4 and 5 is then taken. Each QUID question has 6 possible answers. Each possible answer is attributed a value of 0, 1, 2, 3, 4 or 5 (with the lowest value/severity answers to each question attributed a value of 0, and the highest value/severity answers attributed a value of 5). If the total score is equal to or less than 3, the next step of the algorithm 400 is shown in Figure 4E. If the total score is greater than 3, a score for QUID 4 is checked. If the score for QUID 4 is greater than 1, an initial label of UUI (urgency urinary incontinence) is applied to the patient. If the score for QUID 4 is 1 or less, a score for QUID 5 is checked. If the score for QUID 5 is greater than 1, an initial label of UUI is applied to the patient. If the score for QUID 5 is 1 or less, an initial label of OAB (overactive bladder) is applied to the patient. If an initial label of OAB or UUI is applied, the next step of the algorithm 400 is shown in Figure 4B.

Following an initial label of OAB or UUI being applied to the patient, Figure 4B shows ICIQ OAB 3, 2 and 1 are asked, with all three to be answered. ICIQ OAB 3, 2, and 1 may be used to further refine the initial label of OAB or UUI. If none of the answers to ICIQ OAB 3, 2 and 1 are above a threshold, the next step of the algorithm 400 is shown in Figure 4C. If the answers to one or more of ICIQ OAB 3, 2 and 1 are above the threshold (e.g., sometimes or above for ICIQ OAB 3, > 2 for ICIQ OAB 2, and > 7 for ICIQ OAB 1, although different thresholds may alternatively be used), bother scores for the relevant question(s) are checked. If no bother score is above a threshold (e.g., > 4, although a different threshold may be used), the next step of the algorithm is shown in Figure 4C. If only one bother score is above the threshold, the initial label of OAB or UUI applied to the patient is checked and updated to include the one of urgency, nocturia or frequency that corresponds to the bother score greater than the threshold (e.g., to indicate the predominant symptom associated with the patient's OAB or UUI). If more than one bother score is above the threshold, the bother scores are compared to one another. If the bother scores are not equal, the highest bother score is selected and the initial label of OAB or UUI is checked and updated to include the one of urgency, nocturia or frequency that corresponds to the highest bother score. If the bother scores are equal, the bother scores are prioritised and selected in order of urgency, frequency and nocturia. The bother score above the threshold and corresponding to the highest priority is selected and the initial label of OAB or UUI is checked and updated to include the one of urgency, nocturia and frequency that corresponds to the selected bother score. The next step of the algorithm 400 after updating the initial label applied to the patient is shown in Figure 4F.

In Figure 4C, POPDI 3, 6 and 5 are asked, with all three to be answered. If none of POPDI 3, 6 and 5 are answered yes, the next step of the algorithm 400 is shown in Figure 4D. If one or more of POPDI 3, 6 and 5 are answered yes, the bother scores for POPDI 3, 6 and 5 are checked. If any of the bother scores for POPDI 3, 6 and 5 are above a threshold (e.g., > 2, although a different threshold may be used), the initial label of OAB or UUI is checked and updated to include POP (pelvic organ prolapse). If none of the bother scores for POPDI 3, 6 and 5 are above the threshold, a check is performed to determine if POPDI 5 was answered yes and POPDI 3 and 6 were answered no. If the check is positive, no further questions are asked and an updated label of Overflow VD (void dysfunction) is applied. If the check is negative, the next step of the algorithm 400 is shown in Figure 4D.

In Figure 4D, an age of the patient is requested. If the patient is younger than 60, no further questions are asked. If the patient is 60 or older, SARC-F 1 is asked. If the answer to SARC-F 1 is ≥ 1, no further questions are asked and an updated label of Functional UI (urinary incontinence) is applied. If the answer to SARC-F 1 is 0, the next SARC-F question is asked. If the answer to the subsequent SARC-F question is ≥ 1, no further questions are asked. If the answer to the subsequent SARC-F question is 0, the next SARC-F question is asked, and so on until either the answer to a subsequent SARC-F question is ≥ 1 (in which case no further questions are asked and an updated label of Functional UI is applied) or the answer to all the SARC-F questions is 0 (in which case no further questions are asked and an updated label of Overflow VD is applied).

Following one of the three branches leading to Figure 4E from Figure 4A (see Figure 4A), in Figure 4E QUID 6 is asked. If the answer to QUID 6 is > 1, QUID 4 and 5 are asked, with both to be answered. The scores for QUID 1, 6, 4 and 5 are then taken. If the summed scores for QUID 6, 4 and 5 (which relate to UUI) are greater than the score for QUID 1 (which relates to SUI), an initial label of MUI/UUI (Mixed, urgency urinary incontinence predominant) is applied. If an initial label of MUI/UUI is applied, the next step of the algorithm 400 is shown in Figure 4F. If the summed scores for QUID 6, 4 and 5 are less than the score for QUID 1, an initial label of MUI/SUI (Mixed, stress urinary incontinence predominant) is applied. If an initial label of MUI/SUI is applied, no further questions are asked.

If the answer to QUID 6 is ≤ 1, QUID 3 and 2 are asked, with both to be answered. If the answer to both QUID 3 and 2 is ≤ 1, no further questions are asked and an initial label of Uncertain is applied. If one or both of QUID 3 and 2 are answered > 1, an age of the patient is requested. If the patient is 60 or older, no further questions are asked and a label of SUI ISD (stress urinary incontinence predominant, with risk factors for intrinsic sphincteric deficiency) is applied. If the patient is younger than 60, the patient is asked whether they have undergone prior UI (urinary incontinence) surgery. If the patient has undergone prior UI surgery, no further questions are asked and an initial label of SUI ISD is applied. If the patient has not undergone prior UI surgery, no further questions are asked and an initial label of SUI is applied.

Any question branch shown in Figures 4A to 4E and providing an initial label containing OAB, UUI or MUI/UUI leads to Figure 4F. In Figure 4F, an overall ICIQ OAB score is determined for the ICIQ OAB questions in the respective branch. Each ICIQ OAB question has 5 possible answers. Each possible answer is attributed a value of 0, 1, 2, 3, or 4 (with the lowest value/severity answers to each question attributed a value of 0, and the highest value/severity answers attributed a value of 4), giving a total possible score of 16 across the four questions. Bother scores are not taken into account in determining the overall ICIQ OAB score. If the overall ICIQ OAB score for the questions answered is ≤ 3, no further questions are asked. If the overall ICIQ OAB score is > 3, ICIQ OAB QoL questions are asked. In the algorithm 400, ICIQ OAB QoL 3, 6 to 8, 10, 12 to 14, 19, 21 to 23 and 25 are asked, with all to be answered. Following that, ICIQ OAB QoL 5, 15, 16 and 27 (referred to below as urgency QoL questions) are asked, with all to be answered. The answers to the urgency QoL questions taken together produce a score that is compared to a threshold. Each ICIQ OAB QoL has 6 possible answers (none of the time, a little of the time, some of the time, a good bit of the time, most of the time, all of the time). Each possible answer is attributed a value of 1, 2, 3, 4, 5 or 6 (with the lowest severity answers to each question attributed a value of 1, and the highest severity answers attributed a value of 6). A maximum possible score for the urgency QoL questions taken together is therefore 24. For example, the threshold value is a score of 12 out of 24, although a different threshold value may alternatively be used. Following that, ICIQ OAB QoL 4, 9, 11, 18 and 24 (referred to below as nocturia QoL questions) are asked, with all to be answered. The answers to the nocturia QoL questions taken together produce a score that is compared to a threshold (for example, 15 out of a possible 30). Following that, ICIQ OAB QoL 17 and 20 (referred to below as frequency QoL questions) are asked, with both to be answered. The answers to the frequency QoL questions taken together produce a score that is compared to a threshold (for example, 6 out of a possible 12). Following that, a total score for the ICIQ OAB QoL questions asked is calculated, at which point the branch of the algorithm 400 ends and no further questions are asked. A check is then performed to see if the total score for any of the urgency QoL questions, the nocturia QoL questions and the frequency QoL questions is equal to or above a respective threshold. If no, the label provided prior to answering any ICIQ OAB QoL questions is maintained. If yes, a check is then performed to see how many of the total scores for the urgency QoL questions, nocturia QoL questions and frequency QoL questions are equal to or above the threshold. If the total score for only one of the urgency QoL questions, the nocturia QoL questions and the frequency QoL questions is equal to or above the respective threshold, the label is updated to include the one or urgency, nocturia or frequency. If the total score for more than one of the urgency QoL questions, nocturia QoL questions and frequency QoL questions is equal to or above the respective threshold, the relative total scores for each of the groups of questions are compared. For example, a score of 18 out of 24 (18/24 or 3/4) with respect to the urgency QoL questions is relatively greater than a score of 20 out of 30 (20/30 or 2/3) with respect to the nocturia QoL questions, despite the absolute score being higher for nocturia. If the relative total scores are not equal, the label is updated to include only the one of urgency, frequency and nocturia with the highest relative total score. If the relative total scores are equal for two or more of urgency, frequency and nocturia, the scores are ranked in order of urgency, frequency and nocturia and the label is updated to include the highest ranking one of urgency, frequency and nocturia.

As can be seen from the flowchart or algorithm 400, the processor 305 is configured to present a user with a series of clinically validated questions, relating to one or more pelvic floor dysfunction conditions or bladder conditions, which are most suitable based on answers to one or more preceding questions. In the embodiment shown, the processor 305 is configured to do so using pre-programmed branching logic, for example in accordance with the flowchart 400 described above, although that is not essential. That may enable a determination of one or more pelvic floor dysfunction conditions or bladder conditions with the least number of questions, increasing a sensitivity and specificity of the determination with fewer questions and less irrelevant data.

The processor 305 is configured to provide a determination of one or more conditions, based on the obtained patient questionnaire data, using the machine learning algorithm 315. In the embodiment shown, the machine learning algorithm 315 is or comprises a neural network, although other machine learning algorithm structures may alternatively be used.

Where a branch of the algorithm 400 ends and no further questions are asked, the answers to the question asked in the branch taken are input to the machine learning algorithm. It may also be seen that some branches of the algorithm end with a label (e.g., Overflow VD, Functional UI etc.). In cases where the machine learning algorithm 315 is not available (for example, prior to the machine learning algorithm 315 being trained initially), the labels may act as preliminary diagnoses or initial determinations based on the answers provided to the clinically validated questions in the algorithm 400. The labels may be based on clinical assessment of previous patient answers to condition-specific questionnaires. If the label is followed by a recommendation for a further workup, the preliminary diagnoses or initial determinations may indicate more invasive medical procedures than a questionnaire could be required in order to confirm or provide sufficient information for an accurate diagnosis (for example, urodynamic testing).

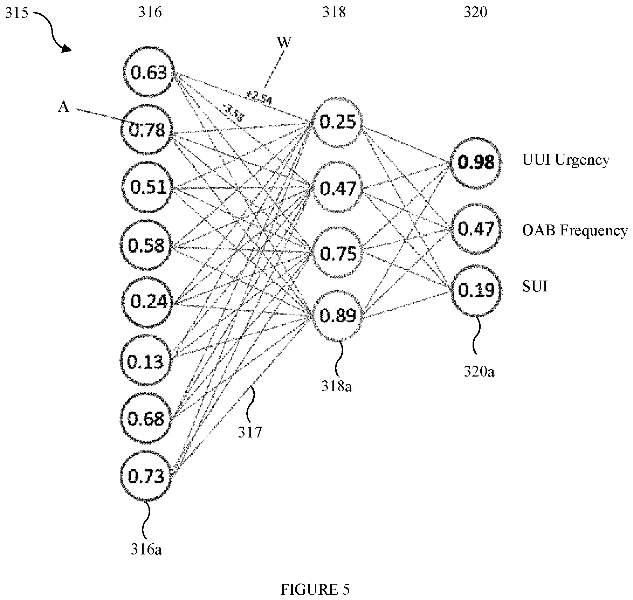

An embodiment of the machine learning algorithm 315 is shown in more detail in Figure 5. In the embodiment shown, the machine learning algorithm 315 is a neural network comprising a plurality of layers, neurons and connections. The neural network comprises an input layer or primary layer 316 comprising a plurality of neurons 316a. Each possible answer to the questions that may be selected by the processor 305 and asked to a user is represented by a separate neuron 316a. The primary layer 316 further comprises additional neurons 316a which represent a behavioural data input (discussed further below), and may also comprise additional neurons 316a which represent a physiological data input (for example, a measured pelvic floor strength of the patient). The number of neurons in the primary layer 316 may vary depending on the number of possible questions and/or behavioural data inputs, possible branching of the questions during selection of a next question by the processor 305 etc. Each neuron 316a is assigned an activation value A of between 0 and 1 (for example, to two decimal places). Neuron activation values are designated by question answers and/or behavioural data inputs which are pre-determined values based on their likelihood of indicating a condition. The specific neuron activation values shown in Figure 5 are for illustrative purposes only, and are not indicative of answers to any questions shown in the algorithm 400 described above.

The primary layer 316 is connected to a secondary or hidden layer 318 by a series of connections 317, although the neural network may alternatively comprise a plurality of secondary or hidden layers 318. The number of hidden layers and the number of neurons in each hidden layer may be determined by a variability of the data set obtained, and each hidden layer 318 may comprise a plurality of neurons 318a. The connections 317 between neurons 316a in the primary layer 316 and neurons 318a in the hidden layer 318 are each assigned a weight W (which may be a positive or negative value) indicating a level of importance of a primary layer neuron 316a to the determination of a condition. The specific weights shown in Figure 5 are for illustrative purposes only. In the embodiment shown, each neuron 316a in the primary layer 316 is connected to each neuron 318a in the hidden layer 318, although that is not essential.

The activation value of a neuron 318a in the hidden layer 318 is then calculated by taking a weighted sum of the activation value for each neuron 316a in the primary layer 316 multiplied by a weight $W$ of its respective connection to the neuron 318a in the hidden layer 318 (e.g., $W1 \cdot A1 + W2 \cdot A2 + W3 \cdot A3 + ... WN \cdot AN$, where A represents the activation value of the Nth neuron 316a in the primary layer 316 and W represents the weight of the connection between the Nth neuron 316a in the primary layer 316 and the neuron 318 of interest in the hidden layer 318).

An activation function is then applied to the weighted sum in order to produce an activation value for the neuron 318a having a value between 0 and 1. In the embodiment shown, the sigmoid function ($S(x) = \frac{1}{1+e^{-x}}$) is used as the activation function, as shown below:

$$S(x) \cdot (W1 \cdot A1 + W2 \cdot A2 + W3 \cdot A3 + \cdots WN \cdot AN)$$

It will be appreciated other activation functions such as the step function, linear function, hyperbolic tangent function, ReLU function, Leaky ReLU function, SoftMax function, Swish self-gated function etc. may alternatively be used.

In order to make the neural network more sensitive, a bias value B may also be added to the above calculation for each neuron 318a in the hidden layer 318, as shown below:

$$S(x) \cdot (W1 \cdot A1 + W2 \cdot A2 + W3 \cdot A3 + \cdots WN \cdot AN + B)$$

The weights $W$ and bias values $B$ are what determines the functionality of the neural network 315 as a whole.

In the embodiment shown, the hidden layer 318 connects to an output layer 320, again through a series of weighted connections as described above. The output layer 320 comprises a plurality of neurons 320a. Each neuron 320a in the output layer 320 represents a determination or classification of a different condition, including but not limited to Stress Urinary Incontinence, Prolapse, Overactive Bladder etc. The number of neurons 320a in the output layer 320 may depend on the how many conditions the neural network is configured or trained to determine. In the embodiment shown, the output layer 320 comprises 14 neurons 320a (although only three are depicted), each representing a different condition - SUI ISD, SUI, MUI/SUI, MUI/UUI, OAB Urgency, UUI Urgency, OAB Nocturia, UUI Nocturia, OAB Frequency, UUI Frequency, OAB POP, UUI POP, Overflow VD, Functional UI. The neuron 320a in the output layer 320 having the highest activation level corresponds to the pelvic floor dysfunction condition or bladder condition the neural network determines the patient is most likely to have. For example, in the example shown in Figure 5, the neuron 320a corresponding to UUI Urgency has the highest activation value, indicating the condition the patient is most likely to have is UUI Urgency.

In addition to questionnaire data, the patient data may include behavioural data. For example, the behavioural data may include metrics such as a volume of fluid drunk (e.g., in L or mL), a volume of fluid urinated (e.g., in L or mL), an amount of caffeine consumed (e.g., in mg), an amount of alcohol consumed (e.g., in units), a number of incontinence episodes, a number of nocturia episodes, a number of bladder voids and a number of incontinence episodes associated with urgency. Each behavioural metric of interest may be represented by a separate neuron 316a in the primary layer 316, as described above in respect of possible question answers.

By structuring the primary layer 316 of the neural network as described above, the neural network 315 is able to provide a determination of one or more pelvic floor dysfunction conditions or bladder conditions based on questionnaire data, behavioural data, or both (and optionally also based on physiological data in conjunction with questionnaire data and/or behavioural data, if available). That may enable the apparatus 300 to provide a determination of one or more conditions even with incomplete patient data or different subsets of data for each patient. In addition, in conjunction with the processor 305 being configured to select questions based on patient data received in response to one or more preceding questions, the apparatus 300 may be configured to improve efficiency and accuracy of the determination by providing the most relevant information for each patient to the neural network 315 (with extraneous information reduced or eliminated).

That may also enable the apparatus 300 to be able to provide an initial determination (for example based on questionnaire data) at a first time, and then subsequently provide an updated or validating determination (for example, based on behavioural data and optionally further/repeat questionnaire data) at a second time. The apparatus 300 may therefore be particularly suitable for longitudinal (for example, repeat) monitoring and determination of one or more pelvic floor dysfunction conditions or bladder conditions.

The neural network 315 is trained to provide a determination, by adjusting the weights and bias values in the neural network 315 in order to achieve accurate determinations. In the embodiment shown, the neural network 315 is trained using backpropagation, with gradient descent used to reduce a value of a cost function, although that is not essential.

The training data used to train the network comprises patient questionnaire data, behavioural data and a corresponding determination or diagnosis for each of a plurality of patients. That may enable the neural network to be trained on a large data set (taking into account outcomes at a population level) in order to increase the accuracy of the determination provided for a new patient. In addition, the training data may be augmented with each new patient for which the apparatus 300 is used to provide a determination. By training the neural network using the patient data received for each new patient together with an external determination or diagnosis (for example, not determined by the apparatus 300) based on that patient data, the neural network may be continually updated and improved based on an increasingly large training dataset over time. In that way, the neural network may be configured to adjust and provide increasingly accurate determinations, rather than maintain a fixed approach as with conventional diagnostic approaches for pelvic floor dysfunction conditions or bladder conditions.

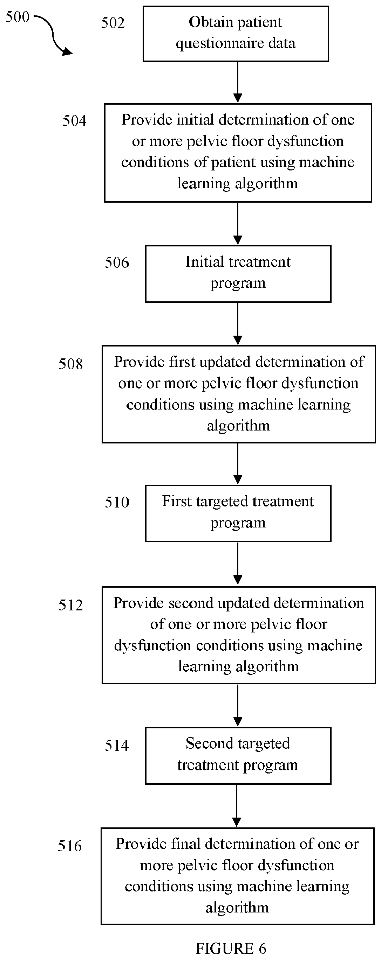

Figure 6 shows a method 500 of determining a pelvic floor dysfunction condition or bladder condition of a patient, in accordance with another embodiment of the present invention. The method 500 may be carried out using, and is described with respect to, the apparatus 300 described above.

Step 502 of the method 500 comprises obtaining or receiving patient questionnaire data, for example using the apparatus 300 as described above with respect to the flowchart 400. The questionnaire data may include personal and/or demographic patient data as well as patient data relating to pelvic floor dysfunction conditions or bladder conditions. The apparatus 300 is configured to generate a digital patient interface (for example, using the processor 305) for a user electronic device, via which a user may input patient data to the apparatus 300, as described above. In the embodiment shown, the apparatus 300 is also configured to generate a digital physician interface (for example, using the processor 305) via which a determination of one or more pelvic floor dysfunction conditions or bladder conditions may be displayed to a physician.

Step 504 of the method 500 comprises providing an initial determination of one or more pelvic floor dysfunction conditions or bladder conditions based on received patient data. The initial determination is provided using a machine learning algorithm, for example via the neural network 315 of the processor 305 as described above.

Step 506 comprises delivering an initial treatment program to the patient. In the embodiment shown, the initial treatment program is delivered to the patient in the form of video and/or audio content via the digital patient interface on a user electronic device. In the embodiment shown, the initial treatment program is generic to any patient, irrespective of which one or more conditions the initial determination relates to. The initial treatment program may be designed to help cultivate patient engagement, and may include tasks, challenges and classes. The initial treatment program is delivered for a first time period. In the embodiment shown, the first time period comprises one week, although that is not essential and the initial treatment program may comprise a shorter or longer duration.

At least a part of the initial treatment program comprises inputting behavioural data of the patient via the digital patient interface (for example, the completion of a bladder diary involving the logging of daily fluids and toilet visits).

Step 508 of the method 500 comprises providing a first updated determination of one or more pelvic floor dysfunction conditions or bladder conditions based on received patient data. The first updated determination is provided using the machine learning algorithm, for example via the neural network 315 of the processor 305 as described above.

In the embodiment shown, the first updated determination is based on behavioural data of the patient provided or input during the initial treatment program. Alternatively, the first updated determination may be based on both the behavioural data of the patient and further questionnaire data. The further questionnaire data may be obtained, for example, using the apparatus 300 as described above with respect to the flowchart 400. However, the further questionnaire data may not include data not directly related to personal and/or demographic patient data, to avoid repeating collection of patient data which will not change.

In either case, the first updated determination may either validate or confirm the initial determination, or provide a different determination, depending on the additional patient data received. For example, the behavioural data (and optionally further questionnaire data) of the patient may be consistent with questionnaire data provided in respect of the initial determination, in which case the first updated determination may validate the initial determination. Alternatively, the behavioural data (and optionally further questionnaire data) may be at least partially inconsistent with the questionnaire data provided in respect of the initial determination, in which case the first updated determination may provide a different, more relevant determination of one or more conditions for the patient.

The flexibility of the neural network 315 to receive questionnaire data and/or behavioural data of the patient may enable the same approach and apparatus 300 to provide an efficient, quick and accurate reassessment of the initial determination. As more patient data is gathered, that may enable the apparatus 300 to respond quickly to new or changing patient data in order to provide an updated determination of one or more pelvic floor dysfunction conditions or bladder conditions, and enable selection of an appropriate and/or treatment program for the patient going forward.

The first updated determination may be displayed to a physician on the digital physician interface as described above. That may significantly reduce a workload of the physician who may otherwise have to make the determination manually based on received patient data, reducing efficiency, speed and potentially accuracy of the determination. Optionally, the processor 305 may be configured to determine a recommended workup (for example, further testing such as urodynamic testing for a determination of SUI ISD, a Pelvic exam for a determination of OAB/UUI POP, Post Void Residual and Uroflowmetry for a determination of Overflow VD, or a Frailty exam for a determination of Functional UI) and/or targeted treatment program corresponding to the one or more conditions of the first updated determination, which may also be displayed on the digital physician interface. That may further reduce a workload of the physician. The raw questionnaire data and/or behavioural data may also be available for display to the physician on the digital physician interface, if required.

Step 510 of the method 500 comprises delivering a first targeted treatment program to the patient corresponding to the first updated determination. The apparatus 300 is configured to determine the first targeted treatment program based on the first updated determination. The first targeted treatment program is delivered to the patient in the form of video and/or audio content via the digital patient interface on a user electronic device, as described above. The first targeted treatment program is delivered for a second time period. In the embodiment shown, the second time period is four weeks, although that is not essential, and the first targeted treatment program may comprise a shorter or longer duration. The second time period of the first targeted treatment program may be or comprise a longer duration than the first time period of the initial treatment program.

The first targeted treatment program comprises treatment content that is relevant to and appropriate for the one or more conditions of the first updated determination. For example, there may be a pool of treatment approaches from which a first targeted treatment program can be constructed for each of the one or more conditions. The pool of treatment approaches comprises a plurality of treatments. The treatments may be combined in different orders and/or combinations to provide a targeted treatment program for each condition. A volume and/or intensity of each selected treatment may also be altered depending on the condition, in order to further tailor the treatment program. Some of the treatments may be universal to all conditions, whilst other treatments may be specific to only a select number of conditions (e.g., one, two, three conditions etc.). That flexibility may enable the first targeted treatment program to be simply and easily tailored to the condition of the first updated determination and provide individualised patient treatment plans if necessary. The combination of treatments and treatment volumes/intensities of the first targeted treatment program may be predetermined for each condition.

For a first targeted treatment program lasting four weeks, the combinations of treatments and/or the volume or intensity of one or more treatments may be different for each week of the four weeks. That may enable the patient to undertake each combination of treatments for long enough to potentially alleviate their symptoms, whilst also making continual progress to increasingly relevant treatments for the determined condition.

The treatments may comprise informational or educational content to help the patient understand more about their condition and place the targeted treatment program in context, for example explaining the background to certain treatment approaches or detail regarding bladder anatomy and/or bladder conditions. The treatments may also comprise challenges or exercises (to be completed by a patient) intended to alleviate symptoms of the one or more conditions of the first updated determination and/or collect further data from the patient (e.g., behavioural data and/or questionnaire data).

At least a part of the first targeted treatment program comprises inputting behavioural data of the patient via the digital patient interface (for example, the completion of a bladder diary involving the logging of daily fluids and toilet visits).

Step 512 of the method 500 comprises providing a second updated determination of one or more pelvic floor dysfunction conditions or bladder conditions based on received patient data. The second updated determination is provided using the machine learning algorithm 315, for example via the neural network of the processor 305 as described above. In the embodiment shown, the second updated determination is based on behavioural data of the patient provided or input during the first targeted treatment program. Alternatively, the second updated determination may be based on both the behavioural data of the patient and further questionnaire data. The further questionnaire data may be obtained, for example, using the apparatus 300 as described above with respect to the flowchart 400. The further questionnaire data may also include answers to PGI-I (Patient Global Impression of Improvement) questions, which provide feedback regarding the patient's impression of how the first targeted treatment program has improved their condition. However, the further questionnaire data may not include data not directly related to personal and/or demographic patient data, to avoid repeating collection of patient data which will not change.

In either case, the second updated determination may either validate or confirm the initial determination or the first updated determination, or provide a different determination, depending on the additional patient data received. For example, the behavioural data (and optionally further questionnaire data) of the patient may indicate the first targeted treatment program has been effective in improving symptoms of the one or more conditions of the first updated determination. The second updated determination may therefore validate the previous determination. Alternatively, the behavioural data (and optionally further questionnaire data) may indicate the first targeted treatment program has not been effective in improving symptoms of the one or more conditions of the first updated determination. The second updated determination may therefore provide a different, more relevant determination of one or more conditions for the patient.

The second updated determination may be displayed to a physician on the digital physician interface as described above, as described above. The processor 305 may also be configured to determine a recommended workup and/or targeted treatment program corresponding to the one or more conditions of the second updated determination, which may also be displayed on the digital physician interface, as described above. The raw questionnaire data and/or behavioural data may also be available for display to the physician on the digital physician interface, if required.

Step 514 of the method 500 comprises delivering a second targeted treatment program to the patient corresponding to the second updated determination. The apparatus 300 is configured to determine the second targeted treatment program based on the second updated determination. The second targeted treatment program is delivered to the patient in the form of video and/or audio content via the digital patient interface on a user electronic device, as described above. The second targeted treatment program is delivered for a third time period. In the embodiment shown, the third time period is four weeks, although that is not essential, and the second targeted treatment program may comprise a shorter or longer duration. The third time period of the second targeted treatment program may be or comprise a longer duration than the first time period of the initial treatment program or the second time period of the first targeted treatment program.

The second targeted treatment program may be constructed in a substantially similar or identical manner to the first targeted treatment program. For example, if the second updated determination is substantially similar or identical to the first updated determination, that may indicate the first updated determination was correct and the first targeted treatment program was appropriate for that patient. It may therefore be beneficial for the patient to continue with the same or a similar treatment approach (for example, progressing to increasingly relevant treatments and/or treatment volumes/intensities) in the second targeted treatment program in order to improve symptoms.

In contrast, if the second updated determination is different from the first updated determination, that may indicate the first updated determination was not correct and the first targeted treatment program was not appropriate for that patient (for example, may not have improved or even worsened the patient's symptoms). It may therefore be beneficial for the patient to switch to a different treatment approach (for example, a different combination of treatments and/or treatment volumes/intensities) more appropriate for the condition(s) of the second updated determination in the second targeted treatment program, in order to improve symptoms.

At least a part of the second targeted treatment program comprises inputting behavioural data of the patient via the digital patient interface (for example, the completion of a bladder diary involving the logging of daily fluids and toilet visits).

Step 516 of the method 500 comprises providing a final determination of one or more pelvic floor dysfunction conditions or bladder conditions based on received patient data. The final determination is provided using the machine learning algorithm, for example via the neural network 315 of the processor 305 as described above. In the embodiment shown, the final determination is based on behavioural data of the patient provided or input during the second targeted treatment program. Alternatively, the final determination may be based on both the behavioural data of the patient and further questionnaire data. The further questionnaire data may be obtained, for example, using the apparatus 300 as described above with respect to the flowchart 400. The further questionnaire data may also include answers to PGI-I (Patient Global Impression of Improvement) questions, which provide feedback regarding the patient's impression of how the first targeted treatment program has improved their condition. However, the further questionnaire data may not include data not directly related to personal and/or demographic patient data, to avoid repeating collection of patient data which will not change.

In either case, the final determination may either validate or confirm the previous determinations, depending on the additional patient data received. For example, the behavioural data (and optionally further questionnaire data) of the patient may be consistent with questionnaire data and behavioural data provided in respect of the previous determinations and may indicate the second targeted treatment program has been effective in improving symptoms of the one or more conditions of the second updated determination, in which case the final determination may validate the previous determinations. Alternatively, the behavioural data (and optionally further questionnaire data) may be at least partially inconsistent with the questionnaire data and behavioural data provided in respect of the previous determinations and may indicate the second targeted treatment program has not been effective in improving symptoms of the one or more conditions of the second updated determination. If so, the final determination may provide a different, more relevant determination of one or more conditions for the patient.

The second updated determination may be displayed to a physician on the digital physician interface as described above, as described above. The processor 305 may also be configured to determine a recommended workup and/or targeted treatment program corresponding to the one or more conditions of the second updated determination, which may also be displayed on the digital physician interface, as described above. The raw questionnaire data and/or behavioural data may also be available for display to the physician on the digital physician interface, if required.

The method 500 described illustrates how the apparatus 300 is configured to enable efficient, streamlined and frequent determinations of one or more pelvic floor dysfunction conditions or bladder condition, thereby improving the speed and accuracy of diagnosis of such conditions. The apparatus 300 also allows efficient data collection that reduces or eliminates unnecessary data (for example using the flowchart 400 and inputting patient data via the digital patient interface). The apparatus 300 also enables quick and accurate determination of one or more conditions using a machine learning algorithm 315 (for example the neural network) provided with the most relevant patient data. The use of a machine learning algorithm such as a neural network also acts to validate subjective patient questionnaire data using both behavioural data of the patient, and population-based data from a large sample set, further increasing accuracy of the determination. The apparatus 300 may allow a physician to react quickly to any changes in patient data to assess if symptoms are improving, stable or worsening (indicating whether the current treatment program is suitable), thereby enabling rapid alteration of treatment if required and increasing the likelihood of halting further progression of the condition(s).

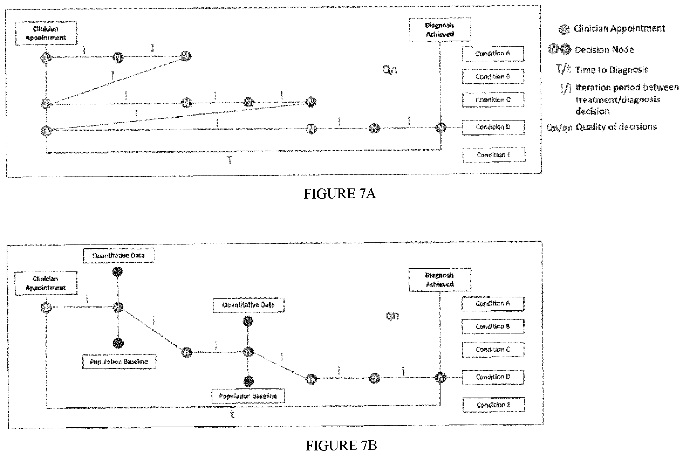

Figures 7A and 7B illustrate the improvement in speed and accuracy of determination of one or more pelvic floor dysfunction conditions or bladder conditions using the apparatus 300 and/or method 500 described above.

Figure 7A shows a conventional pathway for diagnosis and treatment of a pelvic floor dysfunction condition or bladder condition. An initial assessment and determination of a condition is made at a first physician appointment 1, and a first treatment program provided. If the first treatment program is not successful, a modified treatment program is provided for that same condition. If no improvement is made following the modified treatment program, indicating the initial determination was not accurate (which is common, owing to the complexity of diagnosing these conditions), the process begins again at a second physician appointment 2. Because the assessment and determination at each physician appointment is typically made based on questionnaire data alone, an iteration period or time I between each diagnosis and/or treatment decision is often significant (typically months or longer).

Figure 7B shows a pathway for diagnosis and treatment of a pelvic floor dysfunction condition or bladder condition using the apparatus 300 and/or the method 500. An initial assessment and determination of a condition is made a first physician appointment 1, similar to the conventional pathway. However, in contrast to the conventional pathway, each diagnosis and/or treatment decision is based not only on subjective patient questionnaire data but also on population-based data (owing to the use of a trained machine learning algorithm) and behavioural data. That may improve a quality (for example, accuracy or efficiency) of each diagnostic and/or treatment decision compared to the conventional pathway, by validating the patient questionnaire data with two additional data sources. In turn, improved decision quality may enable rapid alteration of a determination and/or treatment approach at different points in time during the treatment process, rather than persisting with a sub-optimal or incorrect determination and treatment approach for an incorrect diagnosis (due to a lack of additional data) and starting the determination and treatment process all over again at a later time.

The pathway of Figure 7B therefore provides the following advantages over the conventional pathway shown in Figure 7A:

- a reduced overall time $t \ll T$ to achieve a correct determination or diagnosis of one or more pelvic floor dysfunction conditions or bladder conditions;
- reduced iteration times $i \ll I$ between diagnostic and/or treatment decisions;
- reduced number $n \ll N$ of diagnostic and/or treatment decisions; and

- a higher decision quality qn>>Qn of diagnostic and/or treatment decisions.

From reading the present disclosure, other variations and modifications will be apparent to the skilled person. Such variations and modifications may involve equivalent and other features which are already known in the art of pelvic floor and bladder dysfunction identification, and which may be used instead of, or in addition to, features already described herein.

Although the appended claims are directed to particular combinations of features, it should be understood that the scope of the disclosure of the present invention also includes any novel feature or any novel combination of features disclosed herein either explicitly or implicitly or any generalisation thereof, whether or not it relates to the same invention as presently claimed in any claim and whether or not it mitigates any or all of the same technical problems as does the present invention.

Features which are described in the context of separate embodiments may also be provided in combination in a single embodiment. Conversely, various features which are, for brevity, described in the context of a single embodiment, may also be provided separately or in any suitable sub-combination. The applicant hereby gives notice that new claims may be formulated to such features and/or combinations of such features during the prosecution of the present application or of any further application derived therefrom.

For the sake of completeness, it is also stated that the term "comprising" does not exclude other elements or steps, the term "a" or "an" does not exclude a plurality, a single processor or other unit may fulfil the functions of several means recited in the claims and any reference signs in the claims shall not be construed as limiting the scope of the claims.

**Claims**

1.  An apparatus for determining a pelvic floor dysfunction condition of a patient, comprising:
    a processor configured to:

    receive patient data, wherein the patient data comprises at least one of questionnaire data and behavioural data of the patient; and
    provide, using a machine learning algorithm, a determination of one or more pelvic floor dysfunction conditions of the patient based on received patient data.

2.  The apparatus of claim 1, wherein the machine learning algorithm is or comprises a neural network.

3.  The apparatus of claim 2, wherein the neural network comprises an input layer comprising a plurality of neurons, each different possible question answer or metric of behavioural data corresponding to a different neuron.

4.  The apparatus of any preceding claim, wherein the machine learning algorithm is configured to:

    provide an initial determination of one or conditions based on questionnaire data received at a first time; and
    provide an updated or validating determination of one or more conditions based on at least behavioural data received at a second time later than the first time, and optionally based on behavioural data and questionnaire data received at the second time.

5.  The apparatus of any preceding claim, wherein the processor is further configured to train or update the machine learning algorithm based on the received patient data.

6.  The apparatus of any preceding claim, further comprising a memory configured to store a plurality of questions relating to a plurality of different conditions; and
    wherein the processor is further configured to:
    select questions relating to one or more conditions for presentation to a user, wherein a next question is selected based on patient data received by the processor in response to one or more preceding questions.

7.  The apparatus of claim 6, wherein:

    the memory is configured to store a ranking for the conditions in order of condition prevalence; and
    the processor is configured to select a first question relating to a most prevalent condition.

8.  The apparatus of claim 6 or of claim 7, wherein:

the processor is configured to:

determine if patient data received in response to one or more preceding questions is indicative of one or more conditions associated with the one or more preceding questions; and

if the patient data is not indicative of a condition associated with the one or more preceding questions, select a next question associated with a next most likely condition.

9. The apparatus of any of claims 6 to 8, wherein the processor is configured to use branching logic to select a next question, and optionally wherein the branching logic is pre-determined.

10. The apparatus of any preceding claim, wherein the behavioural data comprises one or more of a volume of fluid drunk, an amount of caffeine consumed, an amount of alcohol consumed, a number of incontinence episodes, a number of nocturia episodes, a number of bladder voids and a number of incontinence episodes associated with urgency.

11. The apparatus of any preceding claim, wherein the behavioural data is provided for at least one time period, and optionally wherein each time period is 24 hours.

12. The apparatus of any preceding claim, wherein the processor is configured to determine a targeted treatment program based on the determination of the one or more conditions.

13. A method of determining a pelvic floor dysfunction condition of a patient, comprising:

receiving patient data, wherein the patient data comprises at least one of questionnaire data and behavioural data of the patient;

providing, using a machine learning algorithm, a determination of one or more pelvic floor dysfunction conditions of the patient based on received patient data.

14. A non-transitory computer program comprising instructions which, when the program is executed by a processor, cause the processor to carry out the method of claim 13.

15. A computer-readable medium having the computer program of claim 14 stored thereon.

100

```
┌──────────────────────────────────────┐
│                                        │
│   ┌──────────────────────────────┐    │
│   │ 105                          │    │
│   │                              │    │
│   │              ┌─────────┐     │    │
│   │              │  115    │     │    │
│   │              └─────────┘     │    │
│   │                              │    │
│   └──────────────────────────────┘    │
│                                        │
│      ┌─────────┐                       │
│      │  110    │                       │
│      └─────────┘                       │
│                                        │
└──────────────────────────────────────┘
```

FIGURE 1

200

202    ┌────────────────────────┐
       │   Obtain patient data   │
       └────────────────────────┘
                   │
                   ▼
204    ┌────────────────────────────────┐
       │ Determine one or more pelvic floor │
       │ dysfunction conditions of patient  │
       │ using machine learning algorithm   │
       └────────────────────────────────┘

FIGURE 2

300

305

315

325

310

FIGURE 3

400

See Figure 4E

QUID 1 *(SUI)*

> 1

≤ 1

ICIQ OAB 3 *(Urge)*

Never or occasionally

Sometimes or more often

QUID 6 *(Urge + Severity Score)*

QUID 4 *(UUI + Severity Score)*

QUID 5 *(UUI + Severity Score)*

≤ 3

QUID 4, 5, 6 Total Score

> 3

QUID 4 severity score?

≤ 1

QUID 5 severity score?

≤ 1

DX OAB

> 1

> 1

DX UUI

FIGURE 4A

See Figure 4B

FIGURE 4B

FIGURE 4C

FIGURE 4D

```
                                                    ┊
                                                    ▼
         ┌──────────────────┐   > 1   ┌──────────────────┐
         │ QUID 4 (UUI)     │◄────────│ QUID 6 (Urge)    │
         └──────────────────┘         └──────────────────┘
                  │                             │ ≤ 1
                  ▼                             ▼
         ┌──────────────────┐         ┌──────────────────┐  ≤ 1   ┌──────────────────┐
         │ QUID 5 (UUI)     │         │ QUID 3 (SUI)     │───────►│ QUID 2 (ISD)     │
         └──────────────────┘         └──────────────────┘        └──────────────────┘
                  │                             │ > 1               │ ≤ 1      > 1
                  ▼                             ▼                   ▼
  UUI > SUI ┌──────────────────┐      ┌──────────────────┐   ┌──────────────────┐
  ┌─────────│ QUID 1, 6, 4, 5  │      │ QUID 2 (ISD)     │   │ DX Uncertain     │
  │         │ Scores           │      └──────────────────┘   └──────────────────┘
  │         └──────────────────┘               │
  │             SUI > UUI                       │
  ▼                  │                          ▼
┌───────────┐  ┌───────────────┐   ┌─────────┐  < 60  ┌──────────────┐
│ DX MUI/UUI│  │ DX MUI/SUI    │   │ Age     │───────►│ Prior UI     │
└───────────┘  └───────────────┘   └─────────┘        │ Surgery?     │
     ┊                │               │ ≥ 60          └──────────────┘
     ▼                │               ▼          Y          │ N
 See Figure 4F        │         ┌──────────────┐            │
                      │         │ DX SUI ISD   │◄───────────┤
                      │         └──────────────┘            │
                      │               │                     ▼
                      │               │              ┌──────────────┐
                      │               └──────────────│ DX SUI       │
                      ▼                               └──────────────┘
             ┌──────────────────┐
             │ Further workup   │
             │ required         │
             └──────────────────┘
```

FIGURE 4E

FIGURE 4F

315

316          318          320

W

(0.63)

A

(0.78)    +2.54

   -3.58

(0.51)      (0.25)

(0.58)      (0.47)      (0.98)   UUI Urgency

(0.24)      (0.75)      (0.47)   OAB Frequency

(0.13)      (0.89)      (0.19)   SUI

(0.68)     317     318a     320a

(0.73)

316a

FIGURE 5

500

502 — Obtain patient questionnaire data

504 — Provide initial determination of one or more pelvic floor dysfunction conditions of patient using machine learning algorithm

506 — Initial treatment program

508 — Provide first updated determination of one or more pelvic floor dysfunction conditions using machine learning algorithm

510 — First targeted treatment program

512 — Provide second updated determination of one or more pelvic floor dysfunction conditions using machine learning algorithm

514 — Second targeted treatment program

516 — Provide final determination of one or more pelvic floor dysfunction conditions using machine learning algorithm

FIGURE 6

FIGURE 7A

FIGURE 7B

Europäisches
Patentamt

European
Patent Office

Office européen
des brevets

# EUROPEAN SEARCH REPORT

Application Number

EP 22 16 2590

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/280335 A1 (CARLSON DAVID L [US] ET AL) 4 November 2010 (2010-11-04) | 1,13-15 | INV. G16H10/20 G16H50/20 |
| Y | * abstract * * paragraph [0003] - paragraph [0013] * * paragraph [0047] * * paragraph [0073] - paragraph [0089] * | 2-12 | |
| Y | WO 2021/247792 A1 (HEALMED SOLUTIONS LLC [US]) 9 December 2021 (2021-12-09) * abstract; figure 1 * * paragraph [0004] - paragraph [0014] * * paragraph [0054] - paragraph [0110] * | 2-12 | |
| A | MALIK RENA D ET AL: "Domain Comparison Between 6 Validated Questionnaires Administered to Women With Urinary Incontinence", UROLOGY, BELLE MEAD, NJ, US, vol. 132, 13 July 2019 (2019-07-13), pages 75-80, XP085845452, ISSN: 0090-4295, DOI: 10.1016/J.UROLOGY.2019.07.008 [retrieved on 2019-07-13] * abstract * * page 75, column 1, paragraph 1 - page 79, column 2, paragraph 3 * | 1-15 | TECHNICAL FIELDS SEARCHED (IPC) G16H |

The present search report has been drawn up for all claims

| Place of search | Date of completion of the search | Examiner |
|---|---|---|
| Munich | 25 August 2022 | Menschner, Philipp |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 2590

This annex lists the patent family members relating to the patent documents cited in the above-mentioned European search report.
The members are as contained in the European Patent Office EDP file on
The European Patent Office is in no way liable for these particulars which are merely given for the purpose of information.

25-08-2022

| Patent document cited in search report | | Publication date | Patent family member(s) | | Publication date |
|---|---|---|---|---|---|
| US 2010280335 | A1 | 04-11-2010 | CN | 102413871 A | 11-04-2012 |
| | | | CN | 102413872 A | 11-04-2012 |
| | | | CN | 105844087 A | 10-08-2016 |
| | | | EP | 2429643 A1 | 21-03-2012 |
| | | | EP | 2429644 A1 | 21-03-2012 |
| | | | US | 2010280334 A1 | 04-11-2010 |
| | | | US | 2010280335 A1 | 04-11-2010 |
| | | | US | 2010280574 A1 | 04-11-2010 |
| | | | US | 2010280579 A1 | 04-11-2010 |
| | | | WO | 2010126624 A1 | 04-11-2010 |
| | | | WO | 2010126625 A1 | 04-11-2010 |
| WO 2021247792 | A1 | 09-12-2021 | NONE | | |

For more details about this annex : see Official Journal of the European Patent Office, No. 12/82

EPO FORM P0459